# EUROPEAN PATENT SPECIFICATION

(11) **EP 0 856 583 B1**
(45) Date of publication and mention of the grant of the patent: **12.05.2004**
(21) Application number: 98300740.2
(22) Date of filing: 02.02.1998
(51) Int. Cl.: C12N 15/12, C07K 14/47

(54) **Protein rab3 GEP**
Rab3 GEP Protein
Protéine d'échange GDP/GTP (GEP) rab3

(30) Priority: 31.01.1997 JP 1925497
(43) Date of publication of application: 05.08.1998
(73) Proprietor: Japan Science and Technology Corporation, Kawaguchi-shi, Saitama 332 (JP); JCR Pharmaceuticals Co., Ltd., Ashiya-shi, Hyogo (JP)
(72) Inventor: Takai, Yoshimi, Kobe-shi, Hyogo 651-21 (JP); Nakanishi, Hiroyuki, Kobe-shi, Hyogo 651-21 (JP); Wada, Manabu, Kobe-shi, Hyogo 655 (JP)
(74) Representative: Matthews, Derek Peter

(56) References cited:
- CHOW V T K AND LEE S S: "DENN, a novel human gene differentially expressed in normal and neoplastic cells" DNA SEQUENCE, vol. 6, no. 5, 1996, pages 263-273, XP002119552
- DATABASE EMEST16 [Online] EMBL AC C17814, ID HSC8149, 4 October 1996 (1996-10-04) FUJIWARA T ET AL.: "Human placenta cDNA 5'-end GEN-553C12" XP002119556
- ARAKI K ET AL: "Purification and characterization of Rab GDI beta from rat brain." BIOCHEMICAL AND BIOPHYSICAL RESEARCH COMMUNICATIONS, (1995 JUN 6) 211 (1) 296-305. JOURNAL CODE: 9Y8. , XP002119553
- REGAZZI R ET AL.: "Expression, localization and functional role of small GTPases of the Rab3 family in insulin-secreting cells" JOURNAL OF CELL SCIENCE, vol. 109, no. Pt.9, September 1996 (1996-09), pages 2265-2273, XP002119554
- WADA M ET AL: "Isolation and characterization of a GDP/GTP exchange protein specific for the ---Rab3--- subfamily small G proteins." JOURNAL OF BIOLOGICAL CHEMISTRY, (1997 FEB 14) 272 (7) 3875-8. JOURNAL CODE: HIV. , XP002119555

## Description

### Field of the Invention

The present invention relates to a GDP/GTP exchange protein (GEP) specific for the Rab3 subfamily small GTP-binding proteins (G proteins). More particularly, the present invention relates to the protein Rab3 GEP useful for clarification of a molecular mechanism of intracellular vesicle trafficking essential for maintenance of homeostasis of a living organism, or for diagnosis or development of preventive and therapeutic drugs for neural diseases.

### Description of the Related Art

In a general cell composing a living organism, there exist a number of organelles surrounded by unit membranes, such as endoplasmic reticulum, Golgi complex, lysosome, and endosome, and material transport between these organelles is accomplished by accurate trafficking of vesicles (intracellular vesicle trafficking). For instance, membrane receptors, such as EGF and PDGF receptors, are synthesized on ribosomes and transported to the endoplasmic reticulum membrane from where they are transported to the plasma membrane through the Golgi complex by vesicles. Soluble substances, such as those secreted outside the cell from the plasma membrane, are also transported by vesicles. For instance, hormones and digestive enzymes are synthesized on ribosomes and transported to the endoplasmic reticulum lumen from where they are transported to the plasma membrane. Exocytosis, endocytosis, and transcytosis are performed by intracellular vesicle trafficking. There are two exocytotic pathways: one is a regulated pathway and the other is a constitutive pathway. In the former pathway, in most cases exocytosis is regulated by Ca²⁺. Intracellular vesicle trafficking is also involved in various other cell functions, such as formation of cell polarity, cytokinesis and cell motility. Although intracellular vesicle trafficking is one of the very important cellular events as described above, the molecular mechanism has not as yet been completely clarified. The mechanism of intracellular trafficking clarified so far is as follows.

There are at least four principal mechanisms in intracellular vesicle trafficking: (i) budding of the vesicle from the donor membrane; (ii) targetting of the vesicle to the acceptor membrane; (iii) docking of the vesicle to the acceptor membrane; (iv) fusion of the vesicle with the acceptor membrane. The vesicle trafficking is regulated by the Rab family small G proteins. There are approximately thirty members in the Rab family and each member is located in each membrane compartment and exerts its specific function. The mode of action of the Rab family members in the targetting and docking processes in intracellular vesicle trafficking is as follows: the GDP-bound inactive form of each Rab family member is complexed with Rab GDP dissociation inhibitor (GDI) and remains in the cytoplasm. When it is released from Rab GDI, GEP exerts its action, and the Rab family member is converted to the GTP-bound active form. This GTP-bound form binds to its specific target protein on the vesicle, which is consequently transported to the acceptor membrane. Before or after fusion of the vesicle with the membrane, the GTP-bound form is converted to the GDP-bound form. Once the GDP-bound form is produced on the membrane, it is complexed with Rab GDI and translocated from the membrane to the cytoplasm.

On the other hand, the present inventors have discovered Rab3A as a member of the Rab family small G proteins (J. Biol. Chem., 263:2879-2904, 1998), and revealed that Rab3A plays an important role in Ca²⁺-dependent exocytosis, particularly in neurotransmitter release (Int. Rev. Cytol., 133: 187-230, 1992). They have further found Rab GDI, a regulatory protein of Rab3A (J. Boil. Chem. , 265: 2333-2337, 1990) and Rabphilin3A, a target protein of Rab3A (Mol. Cell. Biol., 13: 2061-2068, 1993).

In intracellular vesicle trafficking, as described above, the mode of action of the Rab family members has been clarified, and the research efforts made by the present inventors have permitted specification of regulatory proteins and target proteins of the Rab family members.

However, in order to understand the more detailed mechanism of intracellular vesicle trafficking, it is indispensable to find GEP and GAP specific for each Rab family member or Rab subfamily. At least, no GEP or GAP specific for the Rab3 subfamily members (Rab3A, -B, -C and -D) has not as yet been identified. Two GEPs for Rab3A, Mss4 (Nature, 361: 464-467, 1993) and Rab3A GRF (J. Boil. Chem., 267: 22715-22718, 1992) have been so far found: the former is not specific for the Rab3 subfamily, and the latter has been just partially purified and its primary structure has not been reported.

### Summary of The Invention

The present invention has an object to provide a novel protein (Rab3 GEP) specific for the Rab3 subfamily small G proteins involved in intracellular vesicle trafficking, in a state that the structure (amino acid sequence) and properties thereof have not been clarified.

Another object of the invention is to provide a material for genetic engineering manipulation of this Rab3 GEP.

The invention provides a protein Rab3 GEP, which is a GDP/GTP exchange protein specific for the Rab3 subfamily small G proteins, and comprises the amino acid sequence of Sequence ID No. 1.

In addition, the invention provides a cDNA sequence encoding the amino acid sequence of the Sequence ID No. 1.

The invention also provides a genomic DNA sequence to which the cDNA set forth above or a part thereof is hybridized.

According to the invention, there is provided a novel protein (Rab3 GEP) specific for the Rab3 subfamily small G proteins involved in intracellular vesicle trafficking, and a genetic material for industrially utilizing such a protein. This protein is useful not only for clarifying the molecular mechanism of intracellular vesicle trafficking which is an important cellular event, but also for developing diagnosis, prevention and therapy of neural diseases.

### Brief Description of The Drawings

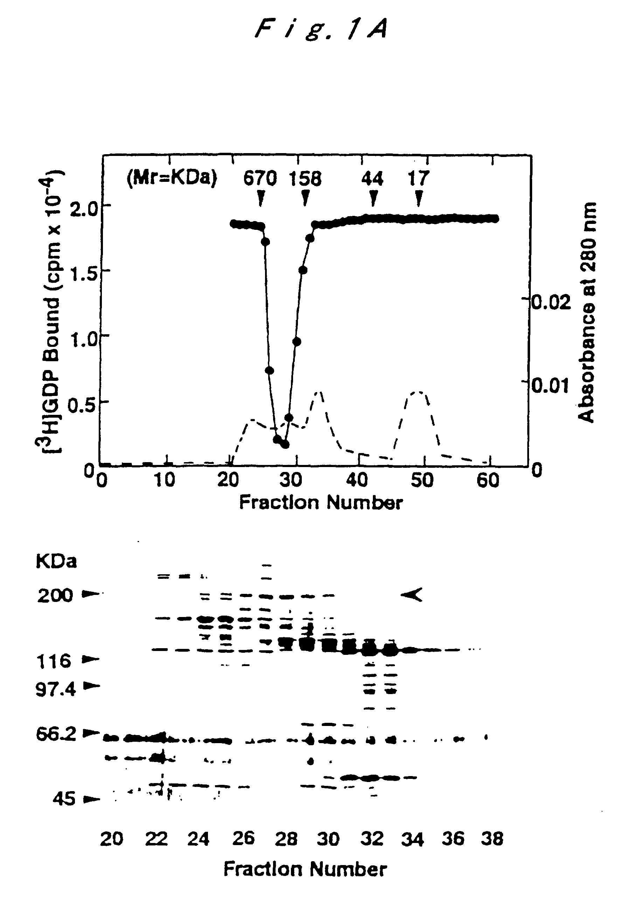

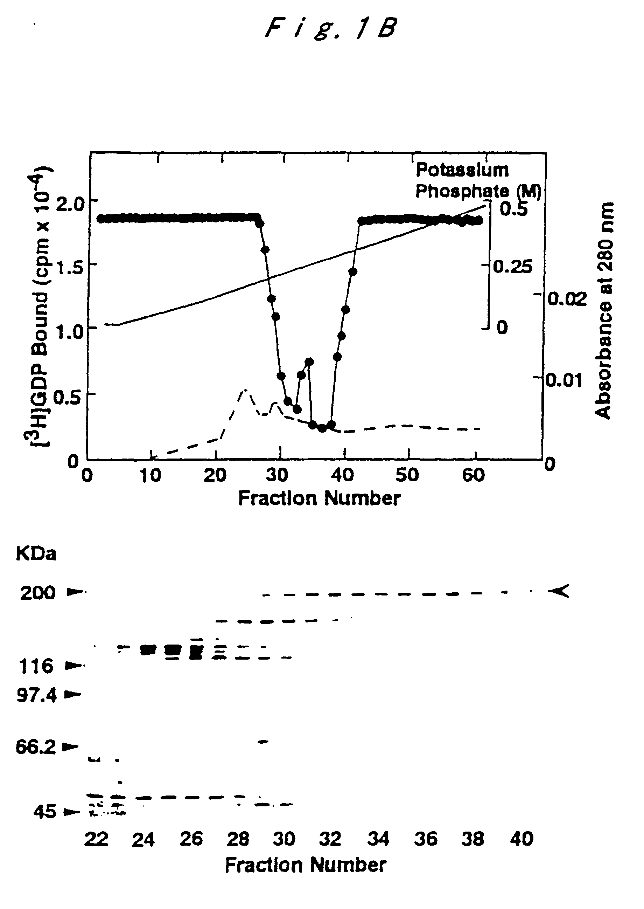

Fig. 1 illustrates the column chromatographies: (A) shows Superdex 200 column chromatography, and (B) shows the second hydroxyapatite column chromatography (● represents the [3H]GDP bound which is an indicator of Rab3 GEP activity, ---, absorbance at 280 nm, and the lower panels illustrate SDS-polyacrylamide gel electrophoresis (PAGE) analysis with silver staining);

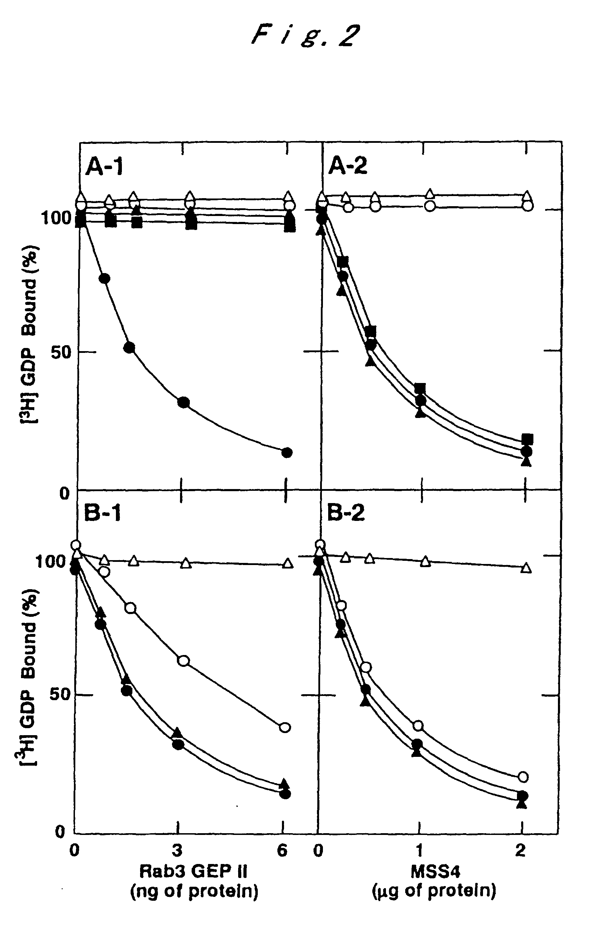

Fig. 2 illustrates the substrate specificity of Rab3 GEP II (A-1, B-1) and Mss4 (A-2, B-2): (A-1, A-2) Rab3A (●), Rab2 (Δ ), Rab5A (○), Rab10 (▲ ) and Rab11 (■ ); and (B-1, B-2) Rab3A (●), Rab3B (Δ ), Rab3C (▲ ), Rab3D (○); and

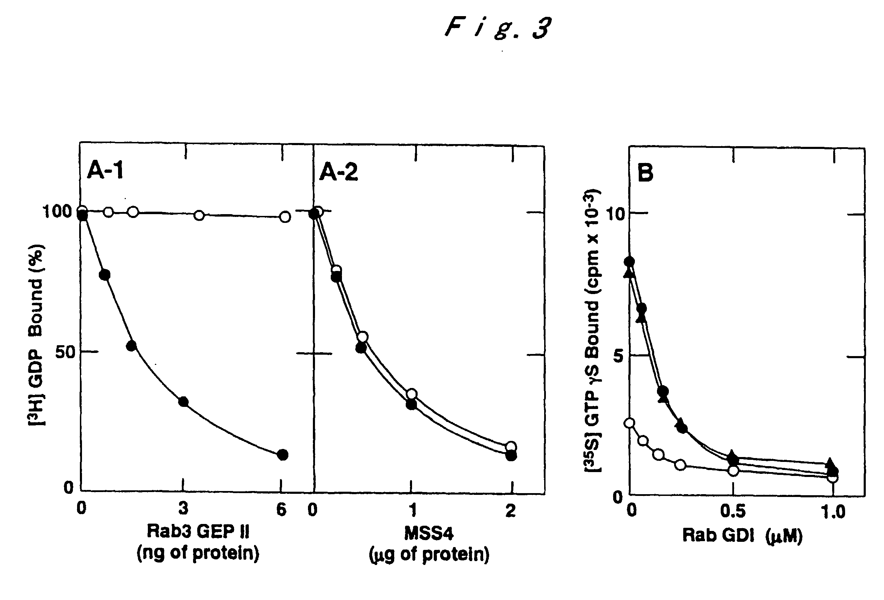

Fig. 3A illustrates the requirement of Rab3 GEP II (A-1) and Mss4 (A-2) for lipid modifications of Rab3A representing their activity to lipid-modified Rab3A (●) and lipid-unmodified Rab3A (○); and Fig. 3B illustrates the sensitivity of Rab3 GEP II and Mss4 to Rab GDI (with Rab3 GEP II (●), with Mss4 (▲ ), without Rab3 GEP II or Mss4 (○))

### Detailed Description of The Invention

A protein Rab3 GEP of the invention is purified from rat brain synaptic soluble fraction through successive column chromatographies by using lipid-modified Rab3A as a substrate, and has a molecular weight of about 200 kD as estimated by SDS-PAGE (about 270 kD as estimated by gel filtration). A cDNA clone was obtained from a rat cDNA library with partial amino acid sequences of this purified protein as probes, and the cDNA amino acid sequence was analyzed. This protein Rab3 GEP was confirmed to have the amino acid sequence of Sequence ID No. 1.

Therefore, Rab3 GEP of the invention is available by inserting the foregoing cDNA into an appropriate expression vector, and expressing the cDNA in Escherichia coli and the like. A protein derived from other animals than rat can be obtained by a process, for example, of isolating a cDNA from the cDNA library of the animal by using the cDNA of the invention or a part thereof as a probe, and causing expression in a suitable host-vector system. The thus obtained protein derived from an animal other than rat also has an amino acid sequence substantially the same as that of Sequence ID No. 1.

The cDNA sequence of the invention includes, as described above, cDNA of rat or cDNA coming from any animal other than rat. The genomic DNA sequence of the invention include DNA sequence of any of all the animal species.

### Examples

A protein Rab3 GEP of the invention will be described further in detail by means of Examples. The invention is not however limited by the following examples.

### Example 1: Purification of Rab3 GEP

Synaptic soluble fraction was prepared from 80 rat brains. A half of the fraction (500 ml, 455 mg of protein) was adjusted to 0.2 M NaCl and applied to a Q-Sepharose FF column (2.6 x 10 cm) equilibrated with Buffer A (20 mM Tris/Cl at pH 7.5 and 1 mM DTT) containing 0.2 M NaCl. Elution was performed with 350 ml of Buffer A containing 0.5 M NaCl. Fractions of 10 ml each were collected. When the Rab3 GEP activity was assayed by measuring the dissociation of [³H]GDP from lipid-modified Rab3A, the activity was observed in Fractions 5-19.

These fractions (150 ml, 159 mg of protein) were collected, and NaCl was added to give a final concentration of 2 M. The sample was applied to a phenyl-Sepharose column (2.6 x 10 cm) equilibrated with Buffer A containing 2 M NaCl. Elution was performed with a 360-ml linear gradient of NaCl (2-0 M) in Buffer A, followed by 180 ml of Buffer A. Fractions of 6 ml each were collected. The Rab3 GEP activity was observed in Fractions 52-63.

These fractions (72 ml, 8. 6 mg of protein) were collected and applied to a hydroxyapatite column (1.0 x 30 cm) equilibrated with Buffer B (20 mM potassium phosphate at pH 7.8, 1 mM DTT, 0.6% CHAPS, and 10% glycerol). Elution was performed with a 75-ml linear gradient of potassium phosphate (20-100 mM) in Buffer B and a subsequent 75-ml linear gradient (100-300 mM) in Buffer B, followed by a 50-ml linear gradient (300-500 mM) in Buffer B. Fractions of 2.5 ml each were collected. The Rab3 GEP activity was observed in Fractions 46-54.

These fractions (22.5 ml, 2.2 mg of protein) were collected, mixed with an equal volume of Buffer C (20 mM bis-Tris/Cl at pH 5.5, 0.5 mM EDTA, 1 mM DTT, 0.6% CHAPS, and 10% glycerol), and applied to a MonoQ HR 10/10 column equilibrated with Buffer C. Elution was performed with a 60-ml linear gradient of NaCl (0.2-0.5 M) in Buffer C. Fractions of 1 ml each were collected. The Rab3 GEP activity was observed in Fractions 24-33.

These fractions (10 ml, 0.44 mg of protein) were collected, concentrated to about 2 ml, and applied to a Superdex 200 column (1.6 x 60 cm) equilibrated with Buffer D (20 mM Tris/Cl at pH 7.5, 0.5 mM EDTA, 1 mM DTT, 0.6 % CHAPS, 0.45% sodium cholate, 10% glycerol, and 0.15 M NaCl). Elution was performed with the same Buffer. Fractions of 2 ml each were collected. The Rab3 GEP activity appeared in Fractions 26-30 (Fig 1A, arrowhead).

These active fractions (10 ml, 45 mg of protein) were collected. The other half of the synaptic soluble fraction was also subjected to the successive column chromatographies in the same manner as described above. The active fractions of the two Superdex 200 column chromatographies were combined and applied to a high pressure liquid chromatography hydroxyapatite column equilibrated with Buffer B. Elution was performed with a 12.5-ml linear gradient of potassium phosphate (20-100 mM) in Buffer B, followed by a 50-ml linear gradient of potassium phosphate (100-500 mM) in Buffer B. Fractions of 1 ml each were collected. The Rab3 GEP activity appeared in two peaks (Fig 1B, arrowhead) in Fraction 29-33 and 34-38 (Fig. 1B). The first (5 ml, 15.5 mg of protein) and second (5 ml, 7.5 mg of protein) peaks were separately collected as Rab3 GEPI and Rab3 GEPII, respectively, and stored at -80°C.

This Rab3 GEPII was found to be inactive on the other Rab families (Rab2, Rab5A, Rab10 and Rab11) (Fig. 2A). Rab3 GEPII was active on Rab3A and Rab3C, partially on Rab3D, but was almost inactive on Rab3B (Fig. 2B). Further, while Rab3 GEPII was active on lipid-modified Rab3A, it was inactive on lipid-unmodified Rab3A (Fig. 3A). These properties of Rab3 GEPII were different from those of protein Mss4 which was equally active on lipid-modified and -unmodified Rab3A and active on many other Rab family members (Figs. 2 and 3) , whereas both Rab3 GEPII and Mss4 were inactive to Rab3A complexed with Rab GDI (Fig. 3B). Rab3 GEPI had almost the same properties as those of Rab3 GEPII.

### Example 2: Peptide mapping of Rab3 GEP and cloning of its cDNA thereof

Rab3 GEPII (20 mg of protein) and Rab3 GEPI (10 mg of protein) purified in the same manner as in Example 1 were separately subjected to SDS-PAGE (6.5% polyacrylamide gel). Each protein band corresponding to a protein with a molecular weight of about 200 kD was isolated from the gel, digested completely with a lysyl endopeptidase, and subjected to C18 reverse phase high pressure liquid chromatography. The sequences of the nine peptides were determined with a peptide sequencer. To determine the N-terminal amino acid sequence of Rab3 GEPII, purified GEPII (4 mg of protein) was applied to SDS-PAGE, and transferred to a PVDF membrane. The protein band was cut from the membrane and directly subjected to the peptide sequencer. A rat brain cDNA library was screened using the oligonucleotide probes designed from the partial amino acid sequences and a cDNA of Rab3 GEPII was cloned. The sequence of this cDNA was determined in accordance with a known method using a DNA sequencer (ABI373), and the amino acid sequence (sequence No. 1) of Rab3 GEPII was determined from the resultant nucleotide sequence.

As a result of homology retrieval by computer, this amino acid sequence exhibited 35% identity with a protein encoded by cDNA yk26g7.5 of Caenorhabditis elegans. While it was almost identical to a protein encoded by human DENN, the human DENN protein lacked about C-terminal 300 amino acids of Rab3 GEP.

### Example 3: Expression of recombinant Rab3 GEP

The cDNA of Rab3 GEP was inserted into the pCMV vector, and the construct was transfected into COS7 cells by the DEAE-dextran method. The COS7 cells were homogenized with a buffer (20 mM Tris/Cl at pH 7.5, 1 mM DTT, and 0.6% CHAPS) and centrifuged at 100,000 x g for 1 hr. The supernatant (2 ml, 4.2 mg of protein) was applied to a Mono Q PC1.6/5 column chromatography. The Rab3 GEP activity of each fraction was measured, and the active fractions were used as recombinant Rab3 GEP.

This recombinant Rab3 GEP had the same properties (requirement for lipid modifications of Rab3A, substrate specificity and sensitivity to Rab GDI) as those of the foregoing purified Rab3 GEPII. Northern blot and Western blot analyses indicated that Rab3 GEP was expressed in all the rat tissues (heart, brain, spleen, lung, liver, skeletal muscle, kidney and testis) with the highest expression in brain.

### Sequence Listing

### Sequence ID No.:1

Length:1602 amino acids
Type: protein
Sequence

Met Val Gln Lys Lys Phe Cys Pro Arg Leu Leu Asp Tyr Leu Val Ile
1               5                   10                  15
Val Gly Ala Arg His Pro Ser Ser Asp Ser Val Ala Gln Thr Pro Glu
            20                  25                  30
Leu Leu Arg Arg Tyr Pro Leu Glu Asp His Pro Glu Phe Pro Leu Pro
        35                  40                  45
Pro Asp Val Val Phe Phe Cys Gln Pro Glu Gly Cys Leu Ser Val Arg
    50                  55                  60
Gln Arg Arg Met Ser Leu Arg Asp Asp Thr Ser Phe Val Phe Thr Leu
65                  70                  75                  80
Thr Asp Lys Asp Thr Gly Val Thr Arg Tyr Gly Ile Cys Val Asn Phe
                85                  90                  95
Tyr Arg Ser Phe Gln Lys Arg Met Pro Lys Glu Lys Ala Glu Gly Gly
            100                 105                 110
Ala Gly Pro Arg Gly Lys Glu Gly Ala His Ala Pro Cys Ala Ser Glu
        115                 120                 125
Glu Ala Ala Thr Glu Ser Ser Glu Ser Gly Ser Thr Leu Gln Pro Pro
    130                 135                 140
Ser Ala Asp Ser Thr Pro Asp Val Asn Gln Ser Pro Arg Gly Lys Arg
145                 150                 155                 160
Arg Ala Lys Ala Gly Asn Arg Ser Arg Asn Ser Thr Leu Thr Ser Leu
                165                 170                 175
Cys Val Leu Ser His Tyr Pro Phe Phe Ser Thr Phe Arg Glu Cys Leu
            180                 185                 190
Tyr Thr Leu Lys Arg Leu Val Asp Cys Cys Ser Glu Arg Leu Leu Gly

        195                 200                 205
Lys Lys Pro Gly Ile Pro Arg Gly Val Gln Arg Asp Thr Met Trp Arg
    210                 215                 220
Ile Phe Thr Gly Ser Leu Leu Val Glu Glu Lys Ser Ser Ala Leu Leu
225                 230                 235                 240
His Asp Leu Arg Glu Ile Glu Ala Trp Ile Tyr Arg Leu Leu Arg Ser
                245                 250                 255
Pro Val Pro Val Ser Gly Gln Lys Arg Val Asp Ile Glu Val Leu Pro
            260                 265                 270
Gln Glu Val Gln Gln Ala Leu Thr Phe Ala Leu Pro Asp Pro Ser Arg
        275                 280                 285
Phe Thr Leu Val Asp Phe Pro Leu His Leu Pro Leu Glu Leu Leu Gly
    290                 295                 300
Val Asp Ala Cys Leu Gln Val Leu Thr Cys Ile Leu Leu Glu His Lys
305                 310                 315                 320
Val Val Leu Gln Ser Arg Asp Tyr Asn Ala Leu Ser Met Ser Val Met
                325                 330                 335
Ala Phe Val Ala Met Ile Tyr Pro Leu Glu Tyr Met Phe Pro Val Ile
            340                 345                 350
Pro Leu Leu Pro Thr Cys Met Ala Ser Ala Glu Gln Leu Leu Leu Ala
        355                 360                 365
Pro Thr Pro Tyr Ile Ile Gly Val Pro Ala Ser Phe Phe Leu Tyr Lys
    370                 375                 380
Leu Asp Phe Lys Met Pro Asp Asp Val Trp Leu Val Asp Leu Asp Ser
385                 390                 395                 400
Asn Arg Val Ile Ala Pro Thr Asn Ala Glu Val Leu Pro Ile Leu Pro
                405                 410                 415
Glu Pro Glu Ser Leu Glu Leu Lys Lys His Leu Lys Gln Ala Leu Ala
            420                 425                 430
Ser Met Ser Leu Asn Thr Gln Pro Ile Leu Asn Leu Glu Lys Phe His

        435                 440                 445
Glu Gly Gln Glu Thr Pro Leu Leu Leu Gly Arg Phe Ser Asn Asp Leu
    450                 455                 460
Gln Ser Thr Pro Ser Thr Glu Phe Asn Pro Leu Ile Tyr Gly Asn Asp
465                 470                 475                 480
Val Asp Ser Val Asp Val Ala Thr Arg Val Ala Met Val Arg Phe Phe
                485                 490                 495
Asn Ser Ala Asn Val Leu Gln Gly Phe Gln Met His Thr Arg Thr Leu
            500                 505                 510
Arg Leu Phe Pro Arg Pro Val Val Ala Phe Gln Ala Gly Ser Phe Leu
        515                 520                 525
Ala Ser Arg Pro Arg Gln Thr Pro Phe Ala Glu Lys Leu Ala Arg Thr
    530                 535                 540
Gln Ala Val Glu Tyr Phe Gly Glu Trp Ile Leu Asn Pro Ser Asn Tyr
545                 550                 555                 560
Ala Phe Gln Arg Ile His Asn Asn Thr Phe Asp Pro Ala Leu Ile Gly
                565                 570                 575
Asp Lys Pro Lys Trp Tyr Ala His Gln Leu Gln Pro Ile His Tyr Arg
            580                 585                 590
Val Tyr Asp Ser Asn Ser Gln Leu Ala Glu Ala Leu Ser Val Pro Pro
        595                 600                 605
Glu Arg Asp Ser Glu Ser Asp Pro Thr Asp Asp Ser Gly Ser Asp Ser
    610                 615                 620
Met Asp Tyr Asp Asp Ser Ser Ser Ser Tyr Ser Ser Leu Gly Asp Phe
625                 630                 635                 640
Val Ser Glu Met Met Lys Cys Asp Ile Asn Gly Asp Thr Pro Asn Val
                645                 650                 655
Asp Pro Leu Thr His Ala Ala Leu Gly Asp Ala Ser Glu Val Glu Ile
            660                 665                 670
Asp Glu Leu Gln Pro Gln Lys Glu Gly Glu Glu Pro Gly Pro Asp Ser

        675                 680                 685
Glu Asn Ser Gln Glu Asn Leu Pro Leu Arg Ser Ser Ser Ser Thr Thr
    690                 695                 700
Ala Ser Ser Ser Pro Ser Thr Ile Val His Gly Ala His Ser Glu Pro
705                 710                 715                 720
Ala Asp Ser Thr Glu Val Gly Asp Lys Ala Ala Thr Gly Ile Ser Lys
                725                 730                 735
Pro Leu Pro Pro Val Pro Pro Ser Ile Cys Lys Ser Thr Val Asp Arg
            740                 745                 750
Arg Gln Thr Glu Thr Gly Glu Gly Ser Val Cys Gln Arg Thr Tyr Asp
        755                 760                 765
His Pro Tyr Phe Glu Pro Gln Tyr Gly Ser Pro Ala Glu Glu Asp Asp
    770                 775                 780
Asp Glu Gln Gly Glu Ser Tyr Thr Pro Arg Phe Ser Gln His Ala Ser
785                 790                 795                 800
Gly Ser Arg Ala Gln Lys Leu Leu Arg Pro Asn Ser Leu Lys Leu Ala
                805                 810                 815
Ser Asp Ser Asp Ala Glu Ser Asp Ser Arg Ala Ser Ser Pro Asn Ser
            820                 825                 830
Thr Val Ser Asn Asn Ser Thr Glu Gly Phe Gly Gly Ile Met Ser Phe
        835                 840                 845
Ala Ser Ser Leu Tyr Arg Asn His Ser Thr Ser Phe Ser Leu Ser Asn
    850                 855                 860
Leu Thr Leu Pro Thr Lys Gly Ala Arg Glu Lys Thr Thr Pro Phe Pro
865                 870                 875                 880
Ser Leu Lys Gly Asn Arg Arg Ala Leu Val Asp Gln Lys Ser Ser Val
                885                 890                 895
Ile Lys His Ser Pro Thr Val Lys Arg Glu Pro Pro Ser Pro Gln Gly
            900                 905                 910
Arg Ser Ser Asn Ser Ser Glu Asn Gln Gln Phe Leu Lys Glu Val Val

        915                 920                 925
His Ser Val Leu Asp Gly Gln Gly Val Gly Trp Leu Asn Met Lys Lys
    930                 935                 940
Val Arg Arg Leu Leu Glu Ser Glu Gln Leu Arg Val Phe Val Leu Ser
945                 950                 955                 960
Lys Leu Ser Arg Ala Val Gln Ser Glu Asp Asp Ala Arg Gln Asp Val
                965                 970                 975
Ile Gln Asp Val Glu Ile Ser Arg Lys Val Tyr Lys Gly Met Leu Asp
            980                 985                 990
Leu Leu Lys Cys Thr Val Leu Ser Leu Glu Gln Ser Tyr Ala His Ala
        995                 1000                1005
Gly Leu Gly Gly Met Ala Ser Ile Phe Gly Leu Leu Glu Ile Ala Gln
    1010                1015                1020
Thr His Tyr Tyr Ser Lys Glu Pro Asp Lys Arg Lys Arg Ser Pro Thr
1025                1030                1035                1040
Glu Asn Val Asn Thr Pro Val Gly Lys Asp Pro Gly Leu Ala Gly Arg
                1045                1050                1055
Gly Asp Pro Lys Ala Met Ala Gln Leu Arg Val Pro Gln Leu Gly Pro
            1060                1065                1070
Arg Ala Pro Ser Ala Thr Gly Arg Gly Pro Lys Glu Leu Asp Thr Arg
        1075                1080                1085
Ser Leu Lys Glu Glu Asn Phe Val Ala Ser Val Gly Pro Glu Val Ile
    1090                1095                1100
Lys Pro Val Phe Asp Leu Gly Glu Thr Glu Glu Lys Lys Ser Gln Ile
1105                1110                1115                1120
Ser Ala Asp Ser Gly Val Ser Leu Ala Ser Ala Ser Gln Arg Thr Asp
                1125                1130                1135
Gln Asp Ser Val Ile Gly Val Ser Pro Ala Val Met Ile Arg Ser Ser
            1140                1145                1150
Ser Gln Asp Ser Glu Val Ser Asn Ser Ser Gly Glu Thr Leu Gly Ala

        1155                1160                1165
Asp Ser Asp Leu Ser Ser Asn Ala Gly Asp Gly Pro Gly Gly Glu Gly
    1170                1175                1180
Ser Ala His Leu Ala Ser Ser Arg Ala Thr Leu Ser Asp Ser Glu Ile
1185                1190                1195                1200
Glu Thr Asn Ser Ala Thr Ser Thr Ile Phe Gly Lys Ala His Ser Leu
                1205                1210                1215
Lys Pro Lys Glu Lys Pro Ala Ser Ser Pro Val Arg Ser Ser Glu Asp
            1220                1225                1230
Val Ser Gln Arg Val Tyr Leu Tyr Glu Gly Leu Leu Gly Arg Asp Lys
        1235                1240                1245
Gly Ser Met Trp Asp Gln Leu Glu Asp Ala Ala Met Glu Thr Phe Ser
    1250                1255                1260
Ile Ser Lys Glu Arg Ser Thr Leu Trp Asp Gln Met Gln Phe Trp Glu
1265                1270                1275                1280
Asp Ala Phe Leu Asp Ala Val Met Leu Glu Arg Glu Gly Met Gly Met
                1285                1290                1295
Asp Gln Gly Pro Gln Glu Met Ile Asp Arg Tyr Leu Ser Leu Gly Glu
            1300                1305                1310
His Asp Arg Lys Arg Leu Glu Asp Asp Glu Asp Arg Leu Leu Ala Thr
        1315                1320                1325
Leu Leu His Asn Leu Ile Ser Tyr Met Leu Leu Met Lys Val Asn Lys
    1330                1335                1340
Asn Asp Ile Arg Lys Lys Val Arg Arg Leu Met Gly Lys Ser His Val
1345                1350                1355                1360
Gly Leu Val Tyr Ser Gln Gln Ile Asn Glu Val Leu Asp Gln Leu Thr
                1365                1370                1375
Asn Leu Asn Gly Arg Asp Leu Ser Ile Arg Ser Ser Gly Ser Arg His
            1380                1385                1390
Met Lys Lys Gln Thr Phe Val Val His Ala Gly Thr Asp Thr Asn Gly

        1395                1400                1405
Asp Ile Phe Phe Met Glu Val Cys Asp Asp Cys Val Val Leu Arg Ser
    1410                1415                1420
Asn Ile Gly Thr Val Tyr Glu Arg Trp Trp Tyr Glu Lys Leu Ile Asn
1425                1430                1435                1440
Met Thr Tyr Cys Pro Lys Thr Lys Val Leu Cys Leu Trp Arg Arg Asn
                1445                1450                1455
Gly Ser Glu Thr Gln Leu Asn Lys Phe Tyr Thr Lys Lys Cys Arg Glu
            1460                1465                1470
Leu Tyr Tyr Cys Val Lys Asp Ser Met Glu Arg Ala Ala Ala Arg Gln
        1475                1480                1485
Gln Ser Ile Lys Pro Gly Pro Glu Leu Gly Gly Glu Phe Pro Val Gln
    1490                1495                1500
Asp Met Lys Thr Gly Glu Gly Gly Leu Leu Gln Val Thr Leu Glu Gly
1505                1510                1515                1520
Ile Asn Leu Lys Phe Met His Asn Gln Val Phe Ile Glu Leu Asn His
                1525                1530                1535
Ile Lys Lys Cys Asn Thr Val Arg Gly Val Phe Val Leu Glu Glu Phe
            1540                1545                1550
Val Pro Glu Ile Lys Glu Val Val Ser His Lys Tyr Lys Thr Pro Met
        1555                1560                1565
Ala His Glu Ile Cys Tyr Ser Val Leu Cys Leu Phe Ser Tyr Val Ala
    1570                1575                1580
Ala Val Arg Ser Ser Glu Glu Asp Leu Arg Thr Pro Pro Arg Pro Val
1585                1590                1595                1600
Ser Ser

## Claims

1. A GDP/GTP exchange protein specific for the Rab3 subfamily G proteins, which comprises the amino acid sequence of Sequence ID No. 1.

2. A cDNA sequence coding the amino acid sequence of the Sequence ID No. 1.

## Patentansprüche

1. Ein GDP/GTP Austauschprotein spezifisch für die G-Proteine der Rab3-Unterfamilie, das die Aminosäuresequenz der Sequenz ID Nr. 1 umfasst.

2. Eine cDNA-Sequenz, welche die Aminosäuresequenz der Sequenz ID Nr. 1 kodiert.

## Revendications

1. Protéine d'échange GDP/GTP spécifique des protéines G de la sous-famille de Rab3, qui comprend la séquence d'acides aminés correspondant à séquence identifiée par le n° 1.

2. Séquence d'ADNc codant pour la séquence d'acides aminés correspondant à la séquence identifiée par le n° 1.
